# DEMANDE DE BREVET EUROPEEN

(11) **EP 2 100 636 A1**
(43) Date de publication de la demande: **16.09.2009**
(21) Numéro de dépôt: 08356043.3
(22) Date de dépôt: 14.03.2008
(51) Int. Cl.: A61M 16/06

(54) **Interface orale de ventilation**

(71) Demandeur: Magnan, Frédéric, 69004 Lyon (FR)
(72) Inventeur: Magnan, Frédéric, 69004 Lyon (FR)

(57) **Abrégé**

Interface orale de ventilation destinée au traitement des apnées du sommeil, possédant un raccord de connexion (4) au tuyau d'alimentation d'air sous pression (16), fixé sur une coque (1) qui passe en pont devant la bouche laissant la cavité buccale libre. Cette coque est supportée par la base de l'interface (2) qui donne insertion à sa face postérieure au joint (3) qui vient se plaquer, de façon étanche, tout autour de la bouche du patient laissant un espace libre entre la partie supérieure de l'interface et la base du nez du patient. Elle possède sur sa coque (1) ou son orifice de raccordement (4) au tuyau d'arrivé d'air sous pression, des orifices qui permettent l'évacuation du gaz carbonique exhalé par le patient.

## Description

Les troubles respiratoires du sommeil, tel que le Syndrome d'Apnée du Sommeil (SAS) sont de plus en plus souvent détectés et traités. Ils se caractérisent en général, par un dysfonctionnement de la fonction respiratoire au cours du sommeil. On observe chez les sujets victimes d'un tel syndrome, une fragmentation importante du sommeil avec des phases de sommeil courtes dues à une succession d'altérations de la mécanique respiratoire et de reprises d'une respiration normale s'accompagnant, le plus souvent, d'un éveil bref de quelques secondes.

Le déroulement normal du sommeil, du stade de sommeil léger au stade de sommeil profond, en passant par un stade de sommeil paradoxal est fortement perturbé, ce qui a des conséquences sur la vie diurne de ces sujets. Ceux-ci ont en effet tendance à être somnolents, voire à s'endormir en pleine activité, et on observe également chez eux une diminution des fonctions intellectuelles et sexuelles ainsi que des risques d'hypoxie, d'hypertension artérielle et d'insuffisance cardiaque. Les troubles respiratoires du sommeil peuvent être de deux types, obstructifs ou centraux.

Les troubles obstructifs représentent la grande majorité des troubles respiratoires nocturnes. On observe, chez ces patients une obstruction totale (apnée obstructive) ou partielle (hypopnée obstructive) des voies aériennes supérieures alors que l'effort musculaire est maintenu. Ce type de trouble est souvent associé à un ronflement important. Les troubles respiratoires obstructifs du sommeil se rencontrent fréquemment chez des sujets obèses, de nombreux arrêts respiratoires nocturnes se produisant par fermeture des voies aériennes supérieures.

De nombreuses études récentes montrent que l'obésité est en nette augmentation dans tous les pays développés, y compris chez l'enfant. De plus, la meilleure connaissance des SAS et la cohorte d'effets secondaires qu'ils entraînent, associée aux progrès réalisés par les nouveaux appareils d'exploration du sommeil, favorisent les dépistages de cette maladie et amènent de plus en plus de sujets à pouvoir bénéficier d'un traitement des SAS.

Le traitement des SAS d'origine obstructive consiste en l'administration d'une Pression Positive Continue (PPC) pendant toutes les périodes de sommeil. Cette PPC permet d'éviter la fermeture des voies aériennes supérieures à l'origine des apnées, elle est soit à un niveau de pression pour les SAS simples, soit à deux niveaux de pression si le patient présente en plus une pathologie associée comme une bronchite pulmonaire chronique obstructive ou une insuffisance respiratoire. Le niveau de cette pression efficace est déterminé par le médecin qui met en place le traitement, elle varie en général entre six et dix-huit centimètres d'eau. Elle est délivrée aux patients par l'intermédiaire d'une interface de ventilation plaquée sur le visage du sujet à l'aide d'un système de fixation amovible. Cette interface doit comporter des orifices permettant l'évacuation hors du masque du gaz carbonique exhalé par le patient.

Les masques connus pour le traitement des SAS par PPC sont principalement de deux types nasal et naso-buccal, mais ils ne donnent pas satisfaction. Ils ne permettent pas de contrôler totalement les fuites qui se produisent entre le masque et le visage du patient, ou par la bouche lorsqu'il s'agit de masque nasal, la pression délivrée dans les voies aériennes supérieures du patient pouvant alors ne plus correspondre à celle prescrite. De taille souvent importante, le champ visuel est perturbé et, associés à leur système de fixation ils sont envahissants sur le visage du patient, ce qui provoque des difficultés de tolérance psychologique. Des douleurs apparaissent souvent au niveau de contact entre le masque et la région péri-nasale, racine du nez ou narines. De plus, les masques nasaux ne peuvent pas être utilisés lorsque le patient présente une obstruction nasale temporaire (rhume) ou définitive (déviation de cloison nasale). Ces différents inconvénients rendent le port quotidien de ces masques durant toutes les périodes nocturnes très difficile, les patients étant fréquemment obligés de retirer le masque de leur visage pendant certaines périodes, ce qui altère l'efficacité du traitement.

La présente invention concerne une interface orale de ventilation et un harnais de fixation qui, seuls ou en combinaison améliorent l'efficacité du traitement des SAS. En effet, contrairement aux habitudes qui consistent à administrer la PPC par le nez, de façon surprenante, son administration orale permet non seulement d'obtenir d'excellents résultats cliniques en terme de disparition des apnées et hypopnées, mais aussi de pallier les différents inconvénients dus aux masques existants.

Etonnamment, la réalisation d'une interface orale et d'un harnais de fixation spécifique, apporte des solutions techniques très appréciables pour le traitement des patients atteints de SAS.

La présente invention consiste en une interface orale passant en pont au dessus de la bouche, destinée à l'administration de PPC pour corriger les troubles des SAS. L'interface comporte des orifices calibrés pour permettre l'évacuation hors du masque du gaz carbonique expiré par le patient. Son joint d'étanchéité s'applique sur le pourtour de la bouche, et permet de conserver un espace libre entre la partie supérieure de l'interface et la partie inférieure du nez du patient. Elle est maintenue en place par un harnais garantissant un maintien efficace tout en étant confortable, peu envahissant sur le visage du patient et simple à mettre en place et à retirer.

Le champ visuel du sujet est totalement libre, il n'y a pas de réaction émotionnelle négative de type claustrophobie altérant l'observance du traitement, car l'interface de la présente invention étant orale, toutes les parties du visage situées au dessus de la base du nez sont totalement dégagées. De plus, dans un exemple préféré de réalisation, l'orifice de raccordement au tuyau d'alimentation en air sous pression étant situé à la partie inférieure de l'interface, le champ visuel reste dégagé même quand le patient regarde en bas, celui-ci a un sentiment de liberté lui faisant pratiquement oublier son masque ; la lecture est alors possible pour favoriser son endormissement s'il le désire.

La petite taille du masque oral, celui-ci n'englobant pas la proéminence nasale, lui permet d'être plus léger, ce qui le rend plus confortable.

Ce masque permet une meilleure tolérance psychologique car il est peu envahissant, il ne couvre qu'une petite partie de la zone inférieure du visage, le patient peut reconnaître sa propre image lorsqu'il se voit dans une glace avec son équipement, et accepte plus facilement d'affronter le regard de son entourage proche.

Conserver ses lunettes, lire ou regarder la télévision pour favoriser son endormissement, sont autant d'avantages rendus possibles car l'interface orale ne nécessite aucune pièce portant un coussinet de contre appui frontal.

L'interface orale est confortable puisqu'elle passe en pont au dessus de la bouche et qu'elle ne possède aucune pièce de bouche, il n'y a pas de phénomène d'hyper salivation engendré par la succion de celle-ci, ni d'intolérance liée à la difficulté de conserver durant toute la période de sommeil une pièce à l'intérieur de la cavité buccale.

Le nez est libre, la racine du nez, zone de configuration très délicate à étancher, n'est pas concernée par l'interface, ce qui supprime le risque de création de fuites habituellement très fréquentes dans cette région et qui, quand elles se produisent en direction des yeux, créent des conjonctivites pouvant nécessiter l'arrêt momentané du traitement.

L'interface orale ne comprime pas la racine du nez et ne provoque pas de douleurs, rougeurs, ou escarres à ce niveau. A la différence des masques existant il n'y a pas de pression prolongée sur cette zone fragile, ce qui provoquerait une hypo vascularisation responsable des troubles sus cités, l'os propre du nez n'étant recouvert que par une fine couche de peau. De plus, la macération ajoutée à l'effet de cisaillement qui se produirait à chaque mouvement respiratoire entre la peau recouvrant le nez et le joint d'étanchéité situé obligatoirement tangentiellement à la peau à cet endroit, favoriseraient l'apparition de ces complications.

Le confort est amélioré par l'interface orale de ventilation car son joint d'étanchéité vient se plaquer sur le pourtour de la bouche, c'est-à-dire sur des parties relativement charnues, ce qui amortit les tensions de serrage du harnais et évite l'apparition de rougeurs et douleurs aux points de contact.

Le masque oral a une excellente efficacité d'élimination du gaz carbonique exhalé par le patient car les orifices calibrés permettant son évacuation à l'extérieur du masque sont très proches de la bouche (à sensiblement moins de trois centimètres de celle-ci), et situés directement sur l'interface. De plus, cette efficacité est également renforcée car le masque a un petit volume interne (moins de quarante millilitres), ce qui réduit l'espace mort au minimum, la quantité d'air vicié à éliminer est donc plus faible.

Un autre avantage du masque est qu'il permet la suppression des risques de congestion nasale ou d'épistaxis entraînant l'arrêt transitoire du traitement car le nez n'est pas traversé par un flux continuel d'air sous pression.

L'interface orale permet d'obtenir toute l'efficacité du traitement même en cas de diminution de la perméabilité nasale transitoire (rhume), ou définitive (déviation de cloison nasale), car le flux d'air sous pression passe exclusivement par la bouche.

Un espace libre est conservé entre la partie supérieure de l'interface orale et la base du nez ce qui permet d'éviter tout contact avec les parties inférieures et internes des narines qui sont des zones fragiles supportant mal l'appui plusieurs heures durant, d'un joint hermétique.

L'administration orale de l'air sous pression est avantageuse pour les patients présentant une déficience d'étanchéité interne entre le compartiment nasal et buccal. Il est en effet aisé de contrôler des fuites qui pourraient se produire alors par le nez, l'adjonction d'un simple pince-nez le faisant efficacement, contrairement à la grande difficulté rencontrée chez ces mêmes patients qui, lorsqu'ils sont ventilés par le nez ont des fuites pratiquement incontrôlables par la bouche, car même la pose d'un bandeau enserrant la tête ne peut empêcher l'air sous pression de s'échapper entre les dents du patient, même si celles-ci sont maintenues serrées.

Grâce au positionnement à la face inférieure de la coque de l'interface du raccord de connexion, le tuyau d'administration d'air sous pression ne crée pas de gêne supplémentaire, car il ne passe pas devant le visage du patient.

Une meilleure stabilité, gage de diminution du risque d'apparition de fuites, est obtenue car l'orifice de raccordement au tuyau d'alimentation d'air sous pression étant placé à la partie inférieure de la coque, le porte-à-faux du système est considérablement réduit.

L'étanchéité et la stabilité du masque sont considérablement améliorées car la souplesse de la base de l'interface autorise la réalisation d'un joint d'étanchéité de faible épaisseur, le joint ainsi formé étant dévolu exclusivement à l'étanchéité, il ne participe pas à l'amortissement des déséquilibres engendrés par les différentes tensions de serrage du harnais de fixation, car ceux-ci sont alors amortis par la souplesse de la base, combinée à celle de la partie avant du harnais. Cette configuration permet d'obtenir une excellente étanchéité, même chez les patients porteurs de barbe ou de moustaches.

L'interface orale ne recouvrant pas le nez, pour couvrir toutes les morphologies adultes, il n'est pas nécessaire de réaliser plusieurs tailles de masque, une seule suffit, car le masque peut déborder plus ou moins autour de la bouche. A l'inverse, les masques existants englobant le nez doivent être fabriqués en de nombreuses tailles différentes pour tenir compte de la proéminence nasale qui varie beaucoup d'un sujet à l'autre.

L'invention concerne également un harnais de fixation totalement nouveau et beaucoup plus performant, permettant de solidariser un masque sur le visage d'un patient.

Les harnais de fixation connus ne permettent pas de répartir la tension de serrage sur l'intégralité de la portée du joint d'étanchéité. Habituellement, les lanières de ces harnais s'accrochent au masque par l'intermédiaire de deux ou quatre points d'encrage fixes, ce qui en cas de déséquilibre de tension entre les différentes sangles occasionne, à cause de la rigidité des masques, une rotation ou un bâillement en inclinaison de ceux-ci, favorisant l'apparition de fuites.

Le harnais de la présente invention épouse par sa partie avant souple, toute la base souple du masque qui porte à sa face postérieure le joint d'étanchéité ; celui-ci est plaqué de façon homogène tout autour de la bouche du patient même en cas de déséquilibre de tension des différentes sangles, car la partie avant du harnais évite les déséquilibres rotatoires de l'interface en tournant légèrement autour de la coque, et sa souplesse combinée à celle de la base, évite les déséquilibres latéraux en ne transmettant pas les forces de décollement à l'autre extrémité du masque.

L'invention concerne donc une interface uniquement orale de ventilation destinée au traitement des apnées du sommeil. Elle possède une coque passant en pont devant la bouche laissant la cavité buccale libre, ainsi qu'un joint qui vient se plaquer de façon étanche tout autour de la bouche du patient, laissant un espace libre entre la partie supérieure de l'interface et la base du nez du patient.

L'interface orale possède des orifices calibrés qui permettent l'évacuation hors du masque, du gaz carbonique exhalé par le patient. Ces orifices sont soit réalisés par le perçage de plusieurs trous ou d'une fente sur sa coque ou son orifice de raccordement, soit obtenus lors de l'emboîtement du raccord de connexion possédant alors des rainures axiales, avec le tuyau d'alimentation d'air pulsé.

Sa coque possède à sa face inférieure ou à sa face avant, un raccord de connexion, droit ou coudé, fixe ou libre en rotation permettant de connecter cette interface au tuyau d'alimentation d'air pulsé sous pression.

Sa coque ou son raccord de connexion au tuyau d'alimentation en air pulsé, comporte au moins un orifice avec bouchon amovible, permettant le raccordement d'appareils de mesure ou de diagnostic, ou l'apport d'oxygène.

La base de sa coque possède deux renflements permettant l'ancrage d'un harnais spécifique de fixation de l'interface orale.

Ce masque est solidarisé au visage du patient par un harnais de fixation spécifique possédant une partie avant souple, de préférence non élastique, évidée en son centre, venant se bloquer derrière les renflements prévus à cet effet sur la coque du masque, et qui se plaque de façon homogène sur l'intégralité de la surface de la base souple portant le joint d'étanchéité. Ce harnais de fixation comporte également deux sangles élastiques horizontales réunis en une de leurs extrémités en une seule partie pouvant, par un système de boucles et de crochets, solidariser ou non cette partie commune à l'extrémité externe de la face avant du harnais. Une languette fixée sur la partie commune des sangles permet de saisir cette partie des sangles. Ces sangles élastiques après avoir fait le tour du crâne du patient pour la sangle supérieure, et le tour de la nuque du patient pour la sangle inférieure, passent dans des anneaux tenus par des lanières fixées à l'autre extrémité de la face avant du harnais, puis viennent s'accrocher chacune sur elle-même, par un système de boucles et de crochets. Ces deux sangles horizontales sont solidarisées par deux brides verticales élastiques, jointes entre elles au niveau de la sangle horizontale inférieure formant ainsi un V, elles coulissent librement sur les deux sangles horizontales.

Dans une variante, sa coque possède des pattes ou des ergots permettant la fixation de l'interface par un harnais.

Les figures illustrent l'invention :

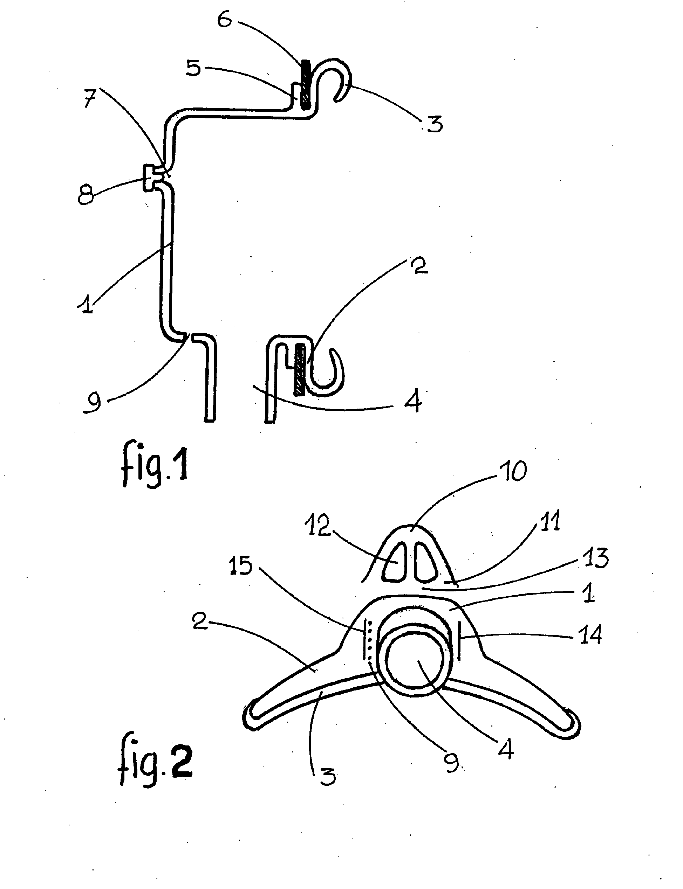

La figure 1, montre une coupe longitudinale du masque.
La figure 2, montre le masque vu de dessous.

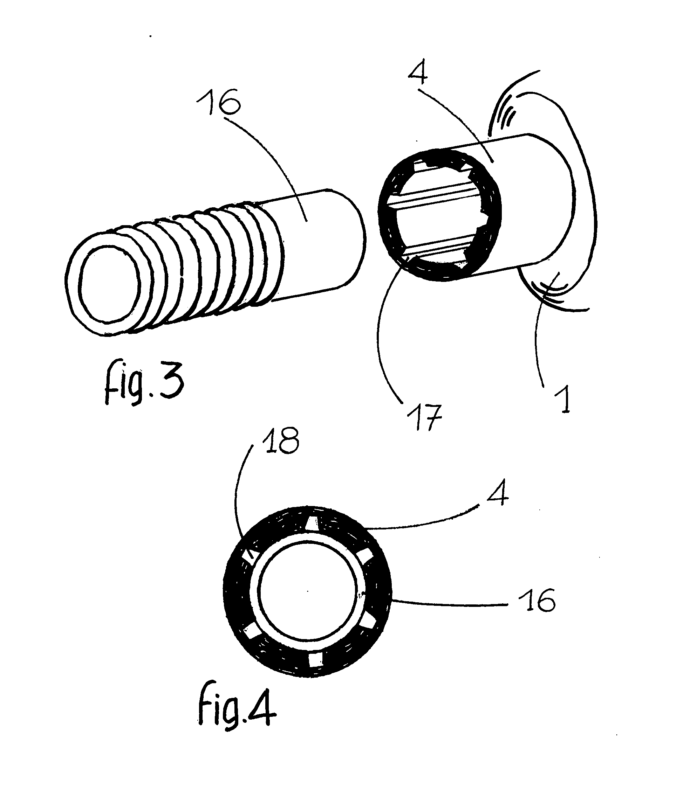

La figure 3, montre les rainures calibrées réalisées dans le raccord de connexion femelle.
La figure 4, montre sur une coupe transversale les orifices réalisés lors de la liaison du raccord de connexion femelle possédant des rainures axiales, avec le tuyau d'alimentation en air.
La figure 5, montre le harnais de fixation de l'interface.

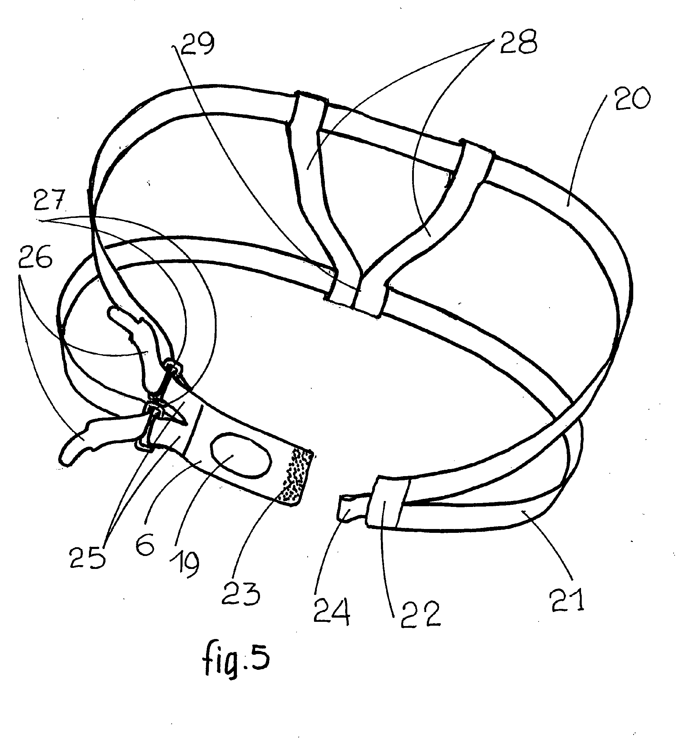

L'interface orale de la présente invention est composée d'une partie avant ou coque (1) portant le raccord de connexion (4) au tuyau d'alimentation en air sous pression (16), et d'une partie arrière appelée la base (2) donnant naissance à sa face inférieure au joint (3) qui vient se plaquer sur le pourtour de la bouche pour assurer l'étanchéité lorsque le masque est solidarisé au visage du patient par l'intermédiaire du harnais de fixation (fig. 5).

Le raccord de connexion (4) est droit ou coudé sensiblement à quatre-vingt-dix degrés, il est fixe ou libre en rotation à trois cent soixante degrés, lorsqu'il est réalisé en plusieurs parties qui s'articulent entre elles. Ce raccord de connexion (4) est placé soit sur la partie avant de la coque (1) dans l'axe de la bouche, soit sur le coté droit ou gauche, mais de préférence à la partie inférieure de la coque (1). Le raccord de connexion (4) est mâle ou femelle, de diamètre standardisé pour se connecter directement au tuyau d'alimentation d'air sous pression (16).

La coque (1) réalisée en matière rigide ou semi-rigide, est transparente ou translucide.

La coque (1) ou le raccord de connexion (4) possède un ou plusieurs orifices (7) permettant soit l'administration d'oxygène, soit le raccordement d'appareils de mesure ou de diagnostic (capteur de pression, thermistance par exemple). Ces orifices (7) sont munis de bouchons amovibles (8) de sorte qu'ils ne créent pas de fuites lorsqu'ils ne sont pas utilisés.

Le joint (3) du masque, vient se plaquer de façon étanche sur le pourtour de la bouche, à l'extérieur des lèvres, sur les zones situées entre le nez (10) et la lèvre supérieure, le menton et la lèvre inférieure, et sur les parties des joues droite et gauche situées à l'extérieur des lèvres. Un espace (13) est gardé libre entre la partie supérieure du joint (3) d'une part, et les narines (12) et la base du nez (11) d'autre part, pour faciliter le confort et la tolérance.

Le masque possède des orifices calibrés destinés à l'évacuation hors du masque du gaz carbonique expiré par le patient. Ce dispositif peut être situé à n'importe quel endroit de la coque (1), cependant la position privilégiée est la présence de ces orifices (9,14) sur la face inférieure de la coque (1), car le désagrément causé par ce flux incessant d'air est ainsi minimisé (si les orifices sont situés sur la face avant par exemple, les flux d'air qui s'en échappent pourraient importuner le patient ou le conjoint éventuel lors de changements de position).

Les flux d'air vicié qui s'échappent de ces orifices sont orientés vers le tuyau d'alimentation d'air (16) par un déflecteur (15) directionnel. Ce déflecteur (15) participe également à l'atténuation du bruit engendré par le passage des flux d'air dans ces orifices.

Dans un exemple de réalisation, ces orifices calibrés sont formés par des trous multiples (9) de petits diamètres de l'ordre d'un millimètre, pour minimiser le plus possible le bruit créé par la sortie de l'air sous pression.

Dans une variante une simple fente calibrée (14), remplace les trous (9) pour laisser échapper le gaz vicié.

Dans un autre exemple de réalisation, ces orifices (18) se réalisent lors de l'assemblage du raccord de connexion (4), qui possède alors des rainures axiales (17), avec le tuyau d'alimentation d'air sous pression (16).

Dans un exemple de réalisation, la coque (1) et le joint (3) sont deux pièces séparées, réalisées en différentes matières. La coque (1) rigide ou semi rigide est fabriquée par exemple en polystyrène, polycarbonate ou polyéthylène téréphtalate. Le joint (3) obligatoirement souple pour pouvoir épouser le pourtour de la bouche, est réalisé de préférence en silicone, gel, mousse, élastomère ou polyuréthane, il est alors solidarisé définitivement à la coque (1) par collage, soudure ou surmoulage par exemple.

Dans une réalisation différente, le joint (3) est assemblé mécaniquement par encliquetage, rainurage ou tout autre moyen approprié ce qui permet de désolidariser ces deux parties en cas de détérioration du joint (3), ou pour faciliter le nettoyage par exemple.

Dans un exemple de réalisation, la coque (1) possède sur sa face avant des ergots permettant l'encrage du harnais de fixation. Deux points d'encrage sont utilisés, ils se trouvent chacun à la partie médiane des extrémités droite et gauche de la face avant de la coque (1). Le système d'attache est alors composé d'une lanière souple et extensible, en tissu élastique ou caoutchouc par exemple, qui vient se fixer sur les ergots du masque par les trous situés à ses extrémités. Plusieurs trous permettent de choisir la tension de serrage adaptée pour obtenir une bonne étanchéité.

Dans un autre exemple, ces ergots sont doublés et situés de chaque coté, aux extrémités inférieure et supérieure droite et gauche de la face avant de la coque (1). La sangle de fixation est alors composée de deux lanières pour renforcer la stabilité du masque.

Dans une variante de réalisation, ces points d'encrage sont des pattes ou anneaux qui laissent le passage aux sangles du harnais, celles-ci après avoir ceinturé ces pattes ou anneaux s'accrochent alors sur elles mêmes par un système de boucles et de crochets.

Dans une réalisation préférée, le masque est fabriqué en une seule pièce et une seule matière comme par exemple du silicone ou tout autre matière possédant des caractéristiques similaires, la rigidité de la coque (1), la souplesse de la base (2) et celle du joint d'étanchéité (3) étant alors obtenues par les variations de forme et d'épaisseur de matière. La dureté de la matière est de quarante à soixante dix shore. La dimension de la coque (1) est réduite au minimum pour diminuer le poids de l'interface et l'espace mort qui est inférieure à quarante millilitres. La base (2) portant la coque (1) sur sa face avant et le joint (3) sur sa face arrière, est souple pour augmenter l'efficacité de l'étanchéité entre l'interface et le pourtour de la bouche du patient, car en combinaison avec la partie avant (6) également souple du harnais spécifique (fig.5) qui vient se plaquer sur toute sa surface, elle absorbe les éventuels déséquilibres de tension de serrage du harnais. Le raccord de connexion (4) au tuyau d'alimentation en air sous pression (16) est situé à la face inférieure de la coque (1), il est femelle, de diamètre standardisé (vingt deux millimètres). Les orifices calibrés permettant l'évacuation hors du masque du gaz carbonique exhalé par le patient, sont réalisés par des rainures axiales (17) dégagées en creux dans l'épaisseur de la face interne du raccord de connexion (4). L'embout du tuyau d'alimentation d'air pulsé (16) n'ayant pas de portée continue, le gaz carbonique rejeté est évacué hors du masque à chaque expiration en passant dans les orifices (18) ainsi créés. Les rainures (17) réalisent, lorsque l'embout du tuyau d'alimentation (16) est emboîté dans le raccord de connexion (4), des orifices (18). Chaque orifice ainsi formé à une section d'un diamètre sensiblement égal à un millimètre et demi. Ainsi situés, ces orifices (18) sont très discrets, et les flux d'air qui s'en échappent ont une direction parallèle au tuyau d'alimentation (16), ils ne se trouvent donc jamais directement dirigés sur la face du patient ou celle de son conjoint éventuel. De plus, le bruit engendré par le passage de l'air est minimisé. L'épaisseur de la coque (1) et celle du raccord de connexion (4) sont sensiblement de trois millimètres et demi, celle de la base (2) sensiblement de trois millimètres, et celle du joint (3) décroissant de sensiblement un millimètre à son point d'encrage à sensiblement un demi millimètre à son extrémité libre. La coque (1) possède à environ trois millimètres de son implantation sur la base (2), deux renflements (5) sur ses faces externes, qui délimitent l'espace permettant de loger et de maintenir en place la partie avant (6) du harnais de fixation (fig.5) spécialement conçu pour ce masque.

La présente invention concerne également un harnais de fixation (fig.5).

Ce harnais innovant, possède un système de libération rapide permettant de désolidariser instantanément, et en un seul geste, le masque du visage du patient. De plus, ce harnais spécifique permet de ne régler qu'une seule fois la tension de serrage de ses sangles, pour chaque patient car ce réglage est indépendant du système d'ouverture et de fermeture du harnais. Lorsque le réglage assurant une étanchéité parfaite du masque tout en conservant un confort optimal est obtenu, celui-ci est conservé pour toutes les séances de ventilations d'un même patient. Ces deux spécificités, permettent d'apporter une plus grande sécurité aux thérapeutes qui peuvent en cas d'urgence, vomissements ou suffocation par exemple, désolidariser en un seul geste le masque du visage du patient. De plus, le fait que le réglage optimal de tension de serrage ne soit effectué qu'une seule fois par patient, augmente l'efficacité de l'interface, évitant que les nombreux thérapeutes intervenant auprès du patient ne fassent des réglages différents les uns des autres.

Les avantages de ce harnais sont également importants pour les patients ventilés à domicile, car la mise en place et le retrait du masque sont grandement facilités, étant réalisés par un simple accrochage, le patient n'a plus à se soucier de l'équilibrage de tension de serrage du masque à chaque séance, celui-ci étant réalisé une seule fois par patient.

Ce harnais se compose d'une partie avant souple, de préférence non élastique (6), et de deux lanières en tissu élastique par exemple (20,21) qui ceinturent le crâne et la nuque du patient. La partie avant (6) comporte en son centre une découpe (19) correspondant à la proéminence de la coque (1) autour de laquelle elle vient se glisser. Elle est maintenue en place par les renflements de matières (5) situés à la partie basale de la coque (1), ce qui lui permet de rester solidaire du masque, même en dehors des périodes de ventilation. Elle est réalisée en un matériaux souple, de préférence non élastique, comme du tissu, plastique, film polyuréthane ou polypropylène transparent par exemple.

Dans la réalisation préférée, cette partie avant (6) est transparente, ce qui augmente la discrétion du système de fixation et le rend mieux supporté par le patient. Cette partie avant (6) possède, à une de ses extrémités le système de fermeture et ouverture rapide du harnais permettant de solidariser ou désolidariser l'extrémité jointe (22) des sangles élastiques horizontales (20,21) à la partie externe de cette face avant (6), cela par accrochage ou encliquetage par exemple.

Dans la réalisation préférée, ce système d'attache est réalisé par l'agrippage d'un système de boucles et de crochets, entre la partie externe (23) de la face avant (6) du harnais et l'extrémité jointe (22) des sangles (20,21), une languette (24) fixée sur cette partie jointe (22) permettant de la saisir. L'autre extrémité de la face avant supporte le système de réglage de la tension des sangles (20,21). Dans la réalisation préférée, deux lanières (25) comportant des anneaux (27) sont cousues, collées, soudées par ultrasons ou agrippées par un système de boucles et de crochets par exemple, sur la partie externe libre de la face avant (6) du harnais. Les sangles (20,21) après avoir entouré le crâne du patient pour la sangle supérieure (20) et la nuque du patient pour la sangle inférieure (21), sont passées dans chaque anneau (27), et viennent s'agripper chacune sur elle-même par un système de boucles et de crochets (26) quand la tension optimum de serrage, alliant étanchéité et confort, est obtenue.

Pour améliorer la stabilité du masque, les deux sangles horizontales élastiques sus décrites (20,21) sont solidarisées entre elles par deux brides verticales (28) également élastiques, qui sont jointes (29) au niveau de la sangle horizontale inférieure (21), décrivant ainsi un V. Ces brides verticales (28) peuvent être déplacées par glissement, vers la droite ou vers la gauche pour ajuster leurs positionnements en fonction de la morphologie de la tête du patient. Elles renforcent l'efficacité du système en évitant l'échappement par le haut de la sangle supérieure (20), qui pourrait se produire chez les patients ayant un sommeil agité.

Les degrés de liberté en rotation autour de la coque (1) que possède la partie avant (6), et la souplesse de cette partie avant (6) en combinaison avec celle de la base (2), permettent de compenser un éventuel déséquilibre dans le serrage des différentes sangles, évitant que le masque lui-même n'en soit affecté.

Pour le bon fonctionnement de l'interface orale de ventilation il faut tout d'abord solidariser le harnais de fixation (fig. 5) au masque en glissant l'orifice (19) de sa partie avant (6), autour de la coque (1) et la bloquer derrière les renflements (5) de celle-ci. Puis on accroche le système de fermeture et ouverture rapide en agrippant le système de boucles et de crochets de la partie commune (22) des sangles horizontales (20,21) sur la partie externe (23) de la face avant du harnais (6). Ensuite on place le masque en pont devant la bouche, en faisant passer la sangle horizontale supérieure (20) autour du crâne et la sangle horizontale inférieure autour de la nuque du patient, on accroche les sangles (20,21) sur elles-mêmes par leur système de boucles et de crochets (26) après les avoir passées dans les anneaux (27) tenus par les lanières (25) fixés à l'autre extrémité externe de la face avant. On solidarise le raccord de connexion (4) au tuyau d'alimentation d'air sous pression (16), on branche le générateur de pression, et enfin, on ajuste les tensions des sangles (20,21) pour que le joint (3) de l'interface se plaque uniformément autour de la bouche assurant l'étanchéité entre le masque et la face du patient. Une fois le bon réglage obtenu, la mise en place et le retrait du masque s'effectue à l'aide de la languette (24) qui permet de solidariser ou désolidariser l'extrémité jointe (22) des sangles élastiques (20,21) à la partie externe (23) de la face avant (6).

A titre d'exemple, l'interface orale de ventilation pour un adulte a une longueur de treize centimètres, une largeur de cinq centimètres et un poids de quarante grammes.

Le harnais fait partie intégrante de l'interface orale de ventilation, il coopère avec le masque et assure une part importante de son étanchéité, de son confort et de sa tolérance par le patient.

Ce harnais innovant est également utilisé pour d'autres masques comportant des systèmes d'accrochages équivalents à celui de l'interface orale de ventilation de la présente invention, les rendant compatibles avec ce harnais.

## Revendications

1. Interface de ventilation destinée au traitement des apnées du sommeil, **caractérisée en ce qu'**elle possède une coque (1) qui passe en pont devant la bouche laissant la cavité buccale libre, qu'elle possède également un joint (3) venant se plaquer de façon étanche tout autour de la bouche du patient en laissant un espace libre (13) entre la partie supérieure de l'interface et la base du nez du patient (11), qu'elle est uniquement orale, et qu'elle possède des orifices pour permettre l'évacuation hors du masque du gaz carbonique exhalé par le patient.

2. Interface orale de ventilation selon la revendication 1 **caractérisée en ce que** sa coque (1) possède à sa face inférieure, un raccord de connexion (4) fixe ou libre en rotation, permettant de connecter cette interface au tuyau d'alimentation d'air pulsé sous pression (16).

3. Interface orale de ventilation selon la revendication 1 **caractérisée en ce que** le raccord de connexion (4) fixe ou libre de rotation, est positionné sur la face avant de la coque (1).

4. Interface orale de ventilation selon l'une des revendications précédentes, **caractérisée en ce que** son raccord de connexion (4) possède des rainures axiales (17) créant lors de l'emboîtement du tuyau d'alimentation d'air pulsé (16) des orifices (18), permettant l'évacuation hors du masque du gaz carbonique exhalé par le patient.

5. Interface orale de ventilation selon l'une des revendications de 1 à 3 **caractérisée en ce que** les orifices d'évacuation du gaz carbonique sont des trous (9) ou une fente (14) situés sur la coque (1) ou sur le raccord de connexion (4).

6. Interface orale de ventilation selon l'une des revendications précédentes **caractérisée en ce que** sa coque (1) ou son raccord de connexion (4) au tuyau d'alimentation d'air pulsé (16) comporte au moins un orifice (7) avec bouchon amovible (8), permettant le raccordement d'appareils de mesure, de diagnostic, ou l'apport d'oxygène.

7. Interface orale de ventilation selon l'une des revendications précédentes, **caractérisée en ce qu'**elle possède à la base de sa coque (1) deux renflements (5) permettant l'ancrage d'un harnais de fixation (fig.5) de l'interface orale.

8. Interface orale de ventilation selon l'une des revendications de 1 à 6 **caractérisée en ce que** sa coque (1) possède des pattes ou des ergots permettant la fixation de l'interface par un harnais.

9. Harnais de fixation d'une interface orale de ventilation selon l'une des revendications de 1 à 7, **caractérisé en ce qu'**il comporte une partie avant souple (6), évidée en son centre (19), venant se bloquer derrière les renflements (5) de la coque (1), et se plaquant de façon homogène sur l'intégralité de la surface de la base (2) souple qui porte le joint d'étanchéité (3).

10. Harnais de fixation d'une interface orale de ventilation selon la revendication 9 **caractérisé en ce qu'**il comporte également deux sangles élastiques horizontales (20,21) réunies à une de leurs extrémités en une seule partie (22) qui par un système de boucles et de crochets est solidarisée ou non à l'extrémité externe (23) de la face avant du harnais (6) par action sur une languette (24), ces sangles élastiques (20,21), après avoir fait le tour du crâne du patient pour la sangle supérieure (20), et le tour de la nuque du patient pour la sangle inférieure (21), passent dans les anneaux (27) tenus par des lanières (25) fixées à l'autre extrémité de la face avant (6), puis viennent s'accrocher chacune sur elle-même, par un système de boucles et de crochets (26), ces deux sangles (20,21) étant solidarisées par deux brides verticales (28) jointes entre elles (29) au niveau de la sangle inférieure (21) formant un V qui coulisse librement sur les sangles horizontales (20,21).
